# EUROPEAN PATENT APPLICATION

(11) **EP 4 130 021 A1**
(43) Date of publication of application: **08.02.2023**
(21) Application number: 21774659.3
(22) Date of filing: 22.03.2021
(51) Int. Cl.: C07K 1/00, A61K 38/02, A61K 38/06, A61K 39/395, A61P 31/16, C07K 1/14, C07K 16/00, C07K 16/28, C07K 16/46, C12N 15/12, C12N 15/62

(54) **METHOD FOR CREATING MULTIMERIC IGA ANTIBODY, AND MULTISPECIFIC MULTIMERIC IGA ANTIBODY**

(30) Priority: 23.03.2020 JP 2020051139
(71) Applicant: Toko Yakuhin Kogyo Co., Ltd., Osaka-shi, Osaka 530-0022 (JP); Japan as Represented by Director-General of National Institute of Infectious Diseases, Tokyo 162-8640 (JP)
(72) Inventor: KAMISHITA, Taizou, Osaka-shi, Osaka 530-0022 (JP); TABATA, Koshiro, Tokyo 162-8640 (JP); SUZUKI, Tadaki, Tokyo 162-8640 (JP); HASEGAWA, Hideki, Tokyo 162-8640 (JP)
(74) Representative: Hoffmann Eitle
(86) International application number: PCT/JP2021/011753
(87) International publication number: WO 2021/193553

(57) **Abstract**

Provided is a method for producing trimeric and tetrameric IgA antibodies, the method including mixing dimeric IgA antibodies and monomeric IgA antibodies.

## Description

### Technical Field

The present invention relates to a method for producing a polymeric IgA antibody, preferably a multispecific polymeric IgA antibody, and the multispecific polymeric IgA antibody.

### Background Art

IgA antibodies are the most-produced antibodies within organisms, and function as a front-line defense factor for biological defense against respiratory infections targeting mucosal tissues such as influenza virus infection. Secretory IgA antibodies (SIgA) form polymers which are dimers or more than dimers. Especially, tetrameric SIgA is known to have higher functional activity than IgG or monomeric IgA.

The present inventors previously developed a technique for producing monoclonal tetrameric SIgA, the technique comprising replacing constant regions other than variable region sequences of a monoclonal IgG antibody by IgA frameworks, and thereby obtaining an IgA-converted antibody while retaining an antigen recognition site of the monoclonal antibody, and then artificially making the antibody change into a secretory and tetrameric type (Patent Literature 1). However, all of eight Fab regions possessed by the tetrameric SIgA produced by the previous technique have the same variable regions, and the tetrameric SIgA has only reactivity to a single epitope.

At present, multispecific antibodies in which two or more Fabs present in the antibody comprise two or more different variable regions have been put into practical use. The multispecific antibodies are expected to be applied not only to antibody drugs but also to various fields including research tools and diagnostic agents. However, methods for producing the multispecific antibodies are complicated, and the production of one multispecific antibody requires a great deal of effort. Thus, only limited types of multispecific antibodies have been put into practical use.

### Citation List

### Patent Literature

Patent Literature 1: Japanese Patent No. 6564777

### Summary of Invention

### Technical Problems

An object of the present invention is to provide a method for producing simply and efficiently a polymeric IgA antibody, particularly a multispecific polymeric IgA antibody.

### Solution to Problems

As a result of extensive research, the present inventors found that bispecific trimeric and tetrameric IgA antibodies were produced simply and efficiently by mixing *in vitro* a dimeric IgA antibody and a monomeric IgA antibody that were each prepared in separate cultured cells, and a secretory component (hereinafter also referred to as "SC"). Thus the present invention was completed. Furthermore, it was found that bispecific trimeric and tetrameric IgA antibodies were produced even when no secretory component was mixed, though the production efficiency was decreased as compared to that when the secretory component was mixed. The techniques of the present invention can be applied to not only production of bispecific trimeric and tetrameric antibodies, but also production of monospecific trimeric and tetrameric antibodies and production of multispecific trimeric and tetrameric antibodies having trispecificity or any specificity more than trispecificity.

Specifically, the present invention provides:
[1] A method for producing trimeric and tetrameric IgA antibodies, the method comprising mixing a dimeric IgA antibody and a monomeric IgA antibody;
[2] The method according to [1], which comprises further mixing a secretory component;
[3] The method according to [1] or [2], wherein the dimeric IgA antibody contains a first antigen binding site and the monomeric IgA antibody contains a second antigen binding site, and the trimeric and tetrameric IgA antibodies have at least bispecificity;
[4] The method according to [3], which comprises further mixing another monomeric IgA antibody containing a third antigen binding site;
[5] The method according to [3], wherein each of four Fab regions of the dimeric IgA antibody contains the first antigen binding site, and each of two Fab regions of the monomeric IgA antibody containing the second antigen binding site contains the second antigen binding site;
[6] The method according to [4], wherein each of four Fab regions of the dimeric IgA antibody contains the first antigen binding site, each of two Fab regions of the monomeric IgA antibody containing the second antigen binding site contains the second antigen binding site, and each of two Fab regions of the another monomeric IgA antibody contains the third antigen binding site;
[7] The method according to any one of [2] to [6], wherein the secretory component is a wild-type SC or a mutant SC;
[8] The method according to any one of [1] to [7], which comprises further mixing at least one substance selected from the group consisting of a molecular chaperone protein, a disulfide bond isomerase, oxidized glutathione, and reduced glutathione;
[9] The method according to any one of [1] to [8], wherein the dimeric IgA antibody and the monomeric IgA antibody are recombinant IgA antibodies separately produced in cultured cells;
[10] The method according to any one of [1] to [9], which further comprises separating the produced trimeric and tetrameric IgA antibodies from other IgA antibodies;
[11] The method according to [10], which further comprises separating the produced trimeric IgA antibody from the produced tetrameric IgA antibody;
[12] A trimeric or tetrameric IgA antibody having at least bispecificity and comprising a first Fab region containing a first antigen binding site and a second Fab region containing a second antigen binding site;
[13] The antibody according to [12], which further comprises a secretory component;
[14] The antibody according to [12], which is a polymer composed of one dimeric IgA antibody molecule containing the first antigen binding site and one or two monomeric IgA antibody molecules containing the second antigen-binding site;
[15] The antibody according to [13], which is a polymer composed of one dimeric IgA antibody molecule containing the first antigen binding site, one or two monomeric IgA antibody molecules containing the second antigen-binding site, and the secretory component;
[16] The antibody according to any one of [12] to [15], which comprises four first Fab regions and at least two second Fab regions;
[17] The antibody according to any one of [13], [15] and [16], wherein the secretory component is a wild-type SC or a mutant SC; and
[18] A pharmaceutical composition comprising the antibody according to any one of [12] to [17].

### Effects of the Invention

The polymeric IgA antibody of the present invention comprises four Fab regions from a dimeric IgA antibody and two or four Fab regions from a monomeric IgA antibody within one antibody molecule, and thus has one type or two or more types of Fab regions. Accordingly, the present invention provides a polymeric IgA antibody, particularly a multispecific polymeric IgA antibody. The polymeric IgA antibody or the multispecific polymeric IgA antibody of the present invention has high antigen-binding or neutralizing activity against each antigen. Further, since polymerization does not occur among dimeric IgA antibodies or monomeric IgA antibodies in the method of the present invention, a trimeric or tetrameric IgA antibody produced by the method of the present invention is a multispecific polymeric antibody with a probability of 100% when a dimeric IgA antibody comprising a first antigen-binding site and a monomeric IgA antibody comprising a second antigen-binding site are used. According to the present invention, a polymeric antibody, particularly a multispecific polymeric antibody, especially a multispecific trimeric or tetrameric antibody, especially a bispecific trimeric or tetrameric antibody is produced by a simple method comprising only mixing *in vitro* at least two types of proteins: a dimeric IgA antibody and a monomeric IgA antibody. The present invention is useful as a platform for development of antibody-based research tools, delivery tools, diagnostic agents, and pharmaceuticals. According to the present invention, the production efficiency of the polymeric antibodies is increased by further subjecting SC to the mixing.

### Brief Description of Drawings

[FIG. 1] Figure 1 is a schematic diagram showing an example of bispecific tetrameric IgA antibody production of the present disclosure.
[FIG. 2-1] Figure 2-1 shows an amino acid sequence of a wild-type SC and an amino acid sequence of a deletion mutant SC12.
[FIG. 2-2] Figure 2-2 shows a nucleotide sequence of a wild-type SC.

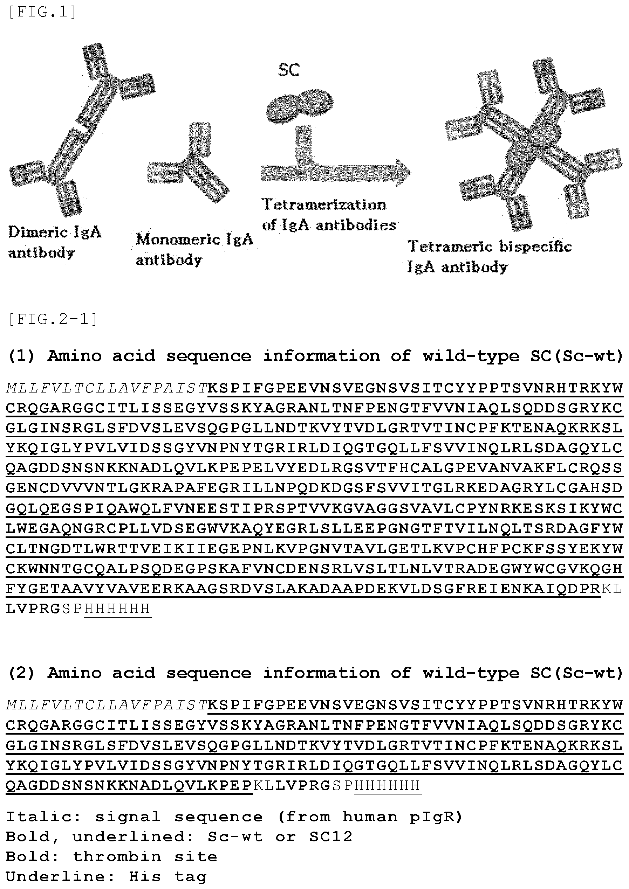

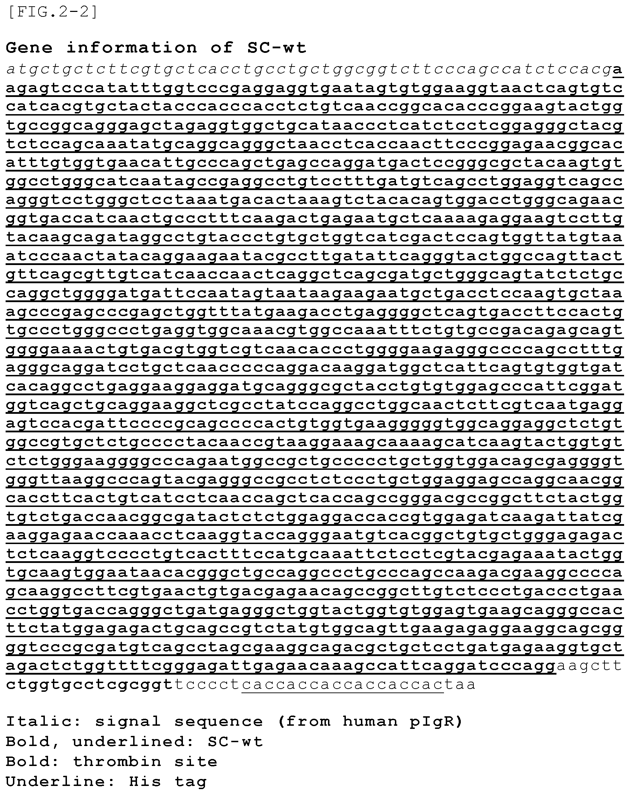

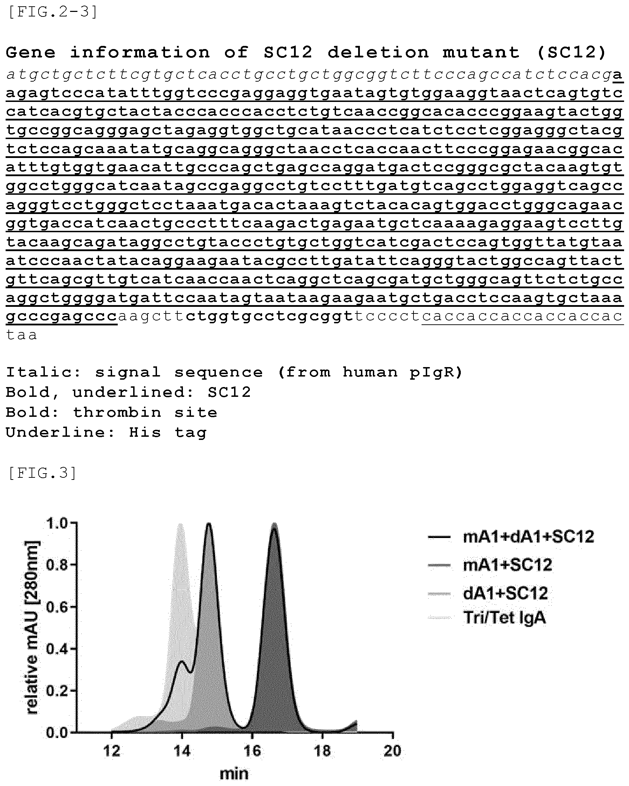

[FIG. 2-3] Figure 2-3 shows a nucleotide sequence of a deletion mutant SC12.
[FIG. 3] Figure 3 is a graph showing the ability of SC12 to promote polymerization of IgA antibodies. In the figure, "mA1+dA1+SC12" (indicated by a solid line) shows a result of mixing of a monomeric IgA1 antibody, a dimeric IgA1 antibody and SC12. "mA1+SC12" shows a result of mixing of a monomeric IgA1 antibody and SC12. "dA1+SC12" shows a result of mixing of a dimeric IgA1 antibody and SC12. "Tri/Tet IgA" shows trimeric and tetrameric IgA1 antibodies produced in cells (control).

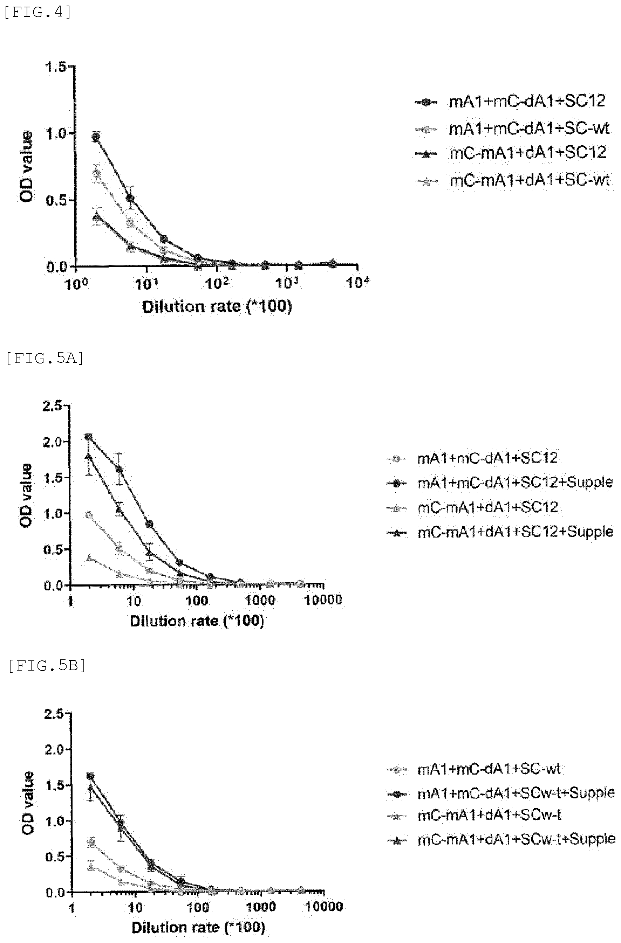

[FIG. 4] Figure 4 is a graph showing a comparison of the ability to promote tetrameric IgA antibody formation between SC12 and SC-wt. In the figure, "mA1+mC-dA1+SC12" shows a result of mixing of a monomeric IgA1 antibody, a fluorescently labeled mCherry-fused dimeric IgA1 antibody, and SC12. "mA1+mC-dA1+SC-wt" shows a result of mixing of a monomeric IgA1 antibody, a fluorescently labeled mCherry-fused dimeric IgA1 antibody, and a wild-type SC. "mC-mA1+dA1+SC12" shows a result of mixing of a fluorescently labeled mCherry-fused monomeric IgA1 antibody, a dimeric IgA1 antibody, and SC12. "mC-mA1+dA1+SC-wt" shows a result of mixing of a fluorescently labeled mCherry-fused monomeric IgA1 antibody, a dimeric IgA1 antibody, and a wild-type SC.
[FIG. 5A] Figure 5A is a graph showing the efficiency of polymeric IgA antibody formation in the presence of SC12 with or without addition of supplements (Supple). In the figure, "mA1+mC-dA1+SC12" shows a result of mixing of a monomeric IgA1 antibody, a fluorescently labeled mCherry-fused dimeric IgA1 antibody, and SC12. "mA1+mC-dA1+SC12+Supple" shows a result of mixing of a monomeric IgA1 antibody, a fluorescently labeled mCherry-fused dimeric IgA1 antibody, SC12, and supplements. "mC-mA1+dA1+SC12" shows a result of mixing of a fluorescently labeled mCherry-fused monomeric IgA1 antibody, a dimeric IgA1 antibody, and SC12. "mC-mA1-dA1+SC12+Supple" shows a result of mixing of a fluorescently labeled mCherry-fused monomeric IgA1 antibody, a dimeric IgA1 antibody, SC12, and supplements.
[FIG. 5B] Figure 5B is a graph showing the efficiency of polymeric IgA antibody formation in the presence of a wild-type SC with or without addition of supplements (Supple). In the figure, "mA1+mC-dA1+SC-wt" shows a result of mixing of a monomeric IgA1 antibody, a fluorescently labeled mCherry-fused dimeric IgA1 antibody, and a wild-type SC. "mA1+mC-dA1+SC-wt+Supple" shows a result of mixing of a monomeric IgA1 antibody, a fluorescently labeled mCherry-fused dimeric IgA1 antibody, a wild-type SC, and supplements. "mC-mA1+dA1+SC-wt" shows a result of mixing of a fluorescently labeled mCherry-fused monomeric IgA1 antibody, a dimeric IgA1 antibody, and a wild-type SC. "mC-mA1+dA1+SC-wt+Supple" shows a result of mixing of a fluorescently labeled mCherry-fused monomeric IgA1 antibody, a dimeric IgA1 antibody, a wild-type SC, and supplements.

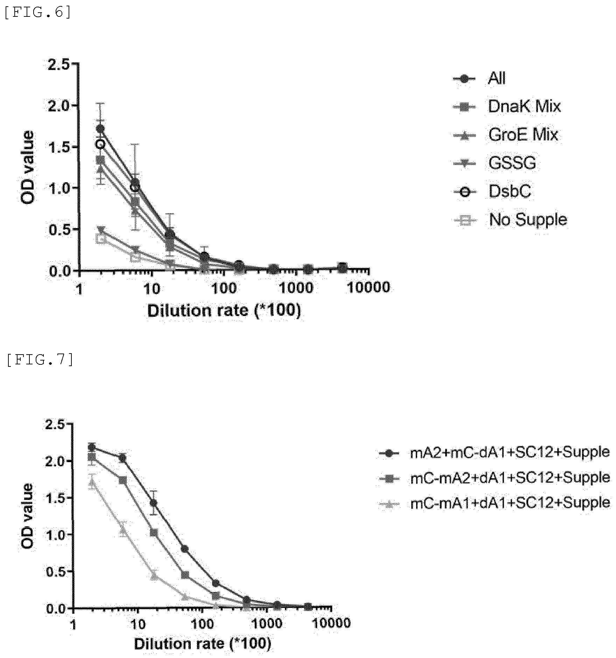

[FIG. 6] Figure 6 is a graph showing an effect of each supplement on promotion of polymeric IgA formation.
[FIG. 7] Figure 7 is a graph showing the polymerization efficiency depending on different IgA antibody constant regions (IgA1 and IgA2m2).

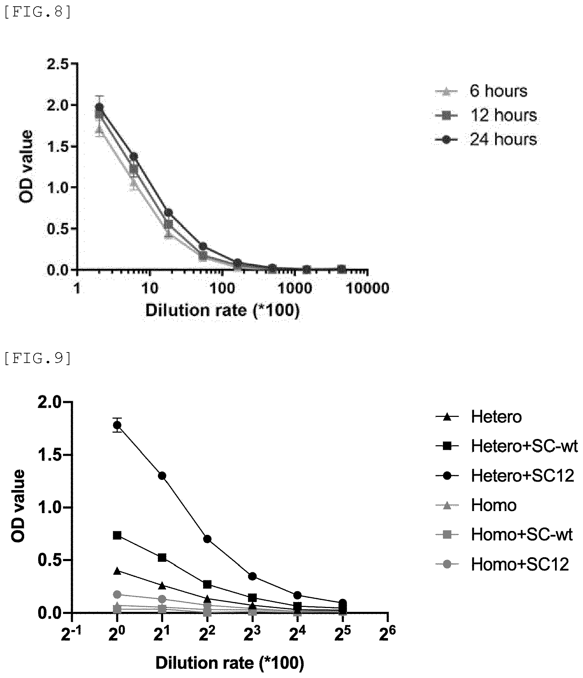

[FIG. 8] Figure 8 is a graph showing the polymerization efficiency depending on different reaction times.
[FIG. 9] Figure 9 is a graph showing the formation efficiency of trimeric and tetrameric IgA antibodies in the presence or absence of SC when using monomeric and dimeric IgA antibodies having two different specificities. In the figure, "Hetero" indicates a combination of a monomeric IgA2m2 antibody (mA2) and a dimeric IgA1 antibody (dA1) having different specificities, and "Homo" indicates a combination of mA2 and dA1 having the same specificity.

### Mode for carrying out the invention

### 1. Terms

Terms used herein have meanings commonly used in the art unless otherwise defined.

The term "antibody" means a protein called an immunoglobulin (Ig), and has the ability to specifically bind to an antigen. Antibodies have a basic unit consisting of four polypeptide chains arranged in a Y-shape that are two heavy (H) chains and two light (L) chains. One or more basic units are assembled to form an antibody molecule. The four polypeptide chains contain constant regions with relatively little variation in amino acid sequences (heavy chain constant regions: CH1, CH2, CH3, and a light chain constant region: CL) and variable regions with large variation in amino acid sequences (a heavy chain variable region: VH, and a light chain variable region: VL).

The term "polymeric" antibody refers to an antibody comprising more than one above-described basic unit. As described later, the antibodies produced by the production method of the present disclosure are trimeric and tetrameric antibodies. Thus, as used herein, the "polymeric antibody" means a trimeric antibody or a tetrameric antibody, or a mixture of a trimeric antibody and a tetrameric antibody.

The term "Fab region" means an antigen-binding site-containing region, consisting of heavy chain VH and CH1 domains and light chain VL and CL domains.

The term "multispecific" antibody means that the antibody can specifically bind to two or more different epitopes (antigenic determinants). The term "bispecific" antibody means that the antibody can specifically bind to two different epitopes. The term "trispecific" antibody means that the antibody can specifically bind to three different epitopes. Similarly, tetraspecific, pentaspecific, hexaspecific, heptaspecific, and octaspecific antibodies mean that the antibodies can specifically bind to four, five, six, seven, and eight different epitopes, respectively. In general, the multispecific antibody comprises two or more antigen binding sites (paratopes) that are each specific for different epitopes. As used herein, the "different epitopes" may be different epitopes on the same antigen or epitopes on different antigens.

The term "monospecific" antibody means that the antibody has one or more antigen binding sites that bind the same epitope on the same antigen.

The term "antigen-binding site" means a region on an antibody molecule which binds to an epitope. The antigen-binding site comprises a heavy chain VH and a light chain VL. As used herein, the first, second, third, and fourth antigen-binding sites means regions consisting of different amino acid sequences to one another which bind to epitopes on different antigens or different epitopes on the same antigen.

"IgA" is one of antibody (immunoglobulin) isotypes. As used herein, IgA is also referred to as "IgA antibody" or "IgA-type antibody". Human IgA is classified into two subclasses: IgA1 and IgA2, depending on different constant regions. IgA2 is further classified into three allotypes: IgA2m1, IgA2m2, and IgA2(n). In a serum, IgA is mainly present as a monomeric IgA (serotype IgA), and IgA1 is the main component. When secreted into mucous membranes, IgA exists as a polymeric IgA (secretory IgA or SIgA) that is a dimer or a higher polymer. IgA2 accounts for approximately half of the polymeric IgA. The polymeric IgA is formed by polymerization of two or more IgA molecules via a J chain (joining chain) or via a J chain and SC. There are the polymeric IgA containing SC and the polymeric IgA not containing SC. The dimeric IgA generally refers to a molecule comprising a heavy chain, a light chain, and a J chain at a ratio of 4:4:1.

The "SC (or secretory component)" is a glycosylated polypeptide having a molecular weight of approximately 70 kDa, and is derived from an extracellular domain of a polymeric immunoglobulin receptor (pIgR). The SC has five immunoglobulin-like domains from the N-terminus, which are designated D1 to D5. Of these, D1 is essential for binding to the polymeric IgA. In D1, there is a structure similar to CDRs (complementarity determining regions) of an immunoglobulin variable region, and such a structure particularly plays an important role in binding to a polymeric IgA.

The plgR is a type I transmembrane protein belonging to an immunoglobulin superfamily, and is expressed on cell membranes of the basal side of mucosal epithelial cells. The plgR is composed of an extracellular domain, a transmembrane domain and an intracytoplasmic domain. The pIgR specifically recognizes and binds a polymeric IgA molecule comprising a J chain which is produced by a plasma cell existing in a mucosal lamina propria, incorporates the polymeric IgA molecule into the epithelial cell, and then transports the polymeric IgA molecule to the apical side while the polymeric IgA molecule is being bound to the pIgR. Then, the pIgR is cleaved between the extracellular domain and the transmembrane domain by a proteolytic enzyme from the epithelial cell, and the polymeric IgA is secreted into a luminal mucosal layer while the polymeric IgA is being bound to the extracellular domain portion of pIgR (secretory IgA or SIgA). The extracellular domain of pIgR after cleavage corresponds to SC. As used herein, an IgA antibody comprising SC in its molecule is referred to as a "secretory IgA antibody".

The "J chain" is a polypeptide having a molecular weight of approximately 15 kDa and having an N-linked sugar chain. The J chain is highly conserved among organisms. The J chain is essential for the polymeric IgA to interact with the pIgR.

### 2. Production method for polymeric IgA antibody

The first aspect of the present invention provides a method for producing trimeric and tetrameric IgA antibodies, and the method comprises mixing a dimeric IgA antibody and a monomeric IgA antibody (hereinafter also referred to as "the production method of the present disclosure"). As a further aspect of the production method of the present disclosure, provided is a method for producing trimeric and tetrameric IgA antibodies, comprising mixing a dimeric IgA antibody, a monomeric IgA antibody, and SC. According to the production method of the present disclosure, one dimeric IgA antibody and two monomeric IgA antibodies are polymerized to produce one tetrameric IgA antibody. Further, according to the production method of the present disclosure, one dimeric IgA antibody, two monomeric IgA antibodies, and SC are polymerized to produce one tetrameric IgA antibody (FIG. 1). Furthermore, according to the production method of the present disclosure, one dimeric IgA antibody and one monomeric IgA antibody are polymerized to produce one trimeric IgA antibody. Furthermore, according to the production method of the present disclosure, one dimeric IgA antibody, one monomeric IgA antibody, and SC are polymerized to produce one trimeric IgA antibody.

The dimeric IgA antibody and the monomeric IgA antibody to be mixed may comprise different variable regions, or may comprise the same variable regions. When all of the variable regions of the dimeric IgA antibody and the monomeric IgA antibody are identical, the resulting trimeric and tetrameric IgA antibodies are monospecific antibodies. When the dimeric IgA antibody and the monomeric IgA antibody comprise two or more different variable regions, multispecific antibodies having bi- to octa-specificity are obtained. For example, a dimeric IgA antibody in which all of the four Fab regions contain a first antigen binding site and a monomeric IgA antibody in which both the two Fab regions contain a second antigen binding site may be used to obtain bispecific trimeric and tetrameric IgA antibodies. For example, a dimeric IgA antibody in which all of the four Fab regions contain a first antigen binding site, a first monomeric IgA antibody in which both the two Fab regions contain a second antigen binding site, and a second monomeric IgA antibody in which both the two Fab regions contain a third antigen binding site may be used to obtain a trispecific tetrameric IgA antibody. For example, a bispecific dimeric IgA antibody in which two of the four Fab regions contain a first antigen binding site and the remaining two Fab regions contain a second antigen binding site, a first monomeric IgA antibody in which both the two Fab regions contain a third antigen binding site, and a second monomeric IgA antibody in which both the two Fab regions contain a fourth antigen binding site may be used to obtain trispecific trimeric IgA antibodies, and trispecific and tetraspecific tetrameric IgA antibodies. Thus, desired multispecific trimeric IgA antibodies having bi- to hexa-specificity and desired multispecific tetrameric IgA antibodies having bi- to octa-specificity can be obtained by changing the variable regions contained in a dimeric IgA antibody and a monomeric IgA antibody to be mixed. Accordingly, in a preferred aspect of the present invention, provided is a method for producing trimeric and tetrameric IgA antibodies having at least bispecificity, which comprises mixing a dimeric IgA antibody comprising a first antigen binding site and a monomeric IgA antibody comprising a second antigen binding site, or mixing a dimeric IgA antibody comprising a first antigen binding site, a monomeric IgA antibody comprising a second antigen binding site, and SC.

In the production method of the present disclosure, polymerization between dimeric IgA antibodies does not occur, and polymerization between monomeric IgA antibodies does not occur. Thus, when a dimeric IgA antibody containing a first antigen-binding site and a monomeric IgA antibody containing a second antigen-binding site are mixed or when a dimeric IgA antibody containing a first antigen-binding site and a monomeric IgA antibody containing a second antigen-binding site and SC are mixed, trimeric and tetrameric IgA antibodies having at least bispecificity are obtained with a probability of 100%.

The dimeric IgA antibody and/or monomeric IgA antibody to be mixed preferably comprises a hydrophobic amino acid residue at position 458 in its heavy chain constant region. Examples of the hydrophobic amino acid include isoleucine, leucine, methionine, tryptophan, and glycine. Isoleucine is preferred. The heavy chain constant region of such an IgA antibody may consist of a natural sequence, or may be altered by a genetic engineering technique to replace the amino acid residue at position 458 with a hydrophobic amino acid. According to the present invention, when an IgA2m2 antibody comprising isoleucine at position 458 in the heavy chain constant region was used, the efficiency of polymeric IgA antibody formation was increased as compared to that when only an IgA1 antibody comprising an amino acid residue other than a hydrophobic amino acid at position 458 in the heavy chain constant region was used (Example 5). The amino acid sequence of the constant region of IgA1 is shown in SEQ ID NO: 1. The amino acid sequence of the constant region of IgA2m2 is shown in SEQ ID NO: 2.

As the SC, either a wild-type SC or a mutant SC may be used. Examples of the mutant SC include a mutant comprising at least domain D1 of SC, for example, a mutant lacking one or more of domains D2 to D5 of SC or lacking a part of the regions of D2 to D5 of SC. Preferred examples of the mutant SC include a mutant comprising at least domains D1 and D2 of SC, a mutant lacking domains D4 and D5 of SC, and a mutant lacking all of domains D3 to D5 of SC (as used herein, referred to as "SC12"). More preferably, SC is a wild-type SC (SEQ ID NO: 3) or SC12 (SEQ ID NO: 4), or a mutant thereof. Still more preferably, SC is SC12 or a mutant thereof. Such a mutant SC may be, for example, a polypeptide consisting of an amino acid sequence having at least 80%, at least 85%, at least 90%, at least 91%, at least 92%, at least 93%, at least 94%, at least 95%, at least 96%, at least 97%, at least 98%, or at least 99% sequence identity to the amino acid sequence shown in SEQ ID NO: 3 or SEQ ID NO: 4. In the production method of the present disclosure, SC may or may not be used, and preferably SC is used. When SC is used, the efficiency of trimeric and/or tetrameric IgA antibody formation is increased.

The origin of the IgA antibodies used in the production method of the present disclosure is not particularly limited. The IgA antibodies may be, for example, human antibodies, non-human mammalian antibodies, rodent-derived antibodies, avian-derived antibodies, or the like.

Antigens to which the IgA antibodies specifically bind are not particularly limited. Appropriate IgA antibodies may be selected to produce the desired trimeric and/or tetrameric IgA antibody.

The subclasses of the IgA antibodies used in the production method of the present disclosure may be either IgA1 or IgA2, or both IgA1 and IgA2. Preferably, both IgA1 and IgA2 may be used. For example, a dimeric IgA1 antibody and a monomeric IgA2 antibody may be used. Further, any allotype of IgA2 may be used.

The IgA antibodies used in the production method of the present disclosure may be natural IgA antibodies or recombinant IgA antibodies. As used herein, the term "recombinant antibody" includes an antibody obtained by altering a natural antibody sequence, and an antibody artificially produced by a genetic engineering technique, regardless of the presence or absence of sequence alteration. For example, a recombinant IgA antibody obtained by converting a non-IgA antibody into an IgA-type may be used. Examples of the non-IgA antibody include an IgG antibody, an IgM antibody, an IgE antibody, an IgD antibody, and an IgY antibody. The origin of the non-IgA antibody is not particularly limited. The non-IgA antibody may be, for example, a human antibody, a non-human mammalian antibody, a rodent-derived antibody, an avian-derived antibody, or the like. For example, the recombinant IgA antibody may be prepared by grafting the variable regions of an IgG antibody into the backbone framework of an IgA antibody, or by grafting only the CDRs of an IgG antibody into the CDRs of an IgA antibody. The recombinant IgA antibody include also a chimeric recombinant IgA antibody comprising a combination of the variable regions of an antibody derived from an animal species and the constant regions of an IgA antibody derived from another animal species, and a recombinant antibody obtained by grafting the complementarity determining regions (CDRs) of an antibody derived from an animal species into the CDRs of an IgA antibody derived from another animal species. Genetic engineering techniques are well known in the art.

Therefore, the term "IgA antibody" as used herein means an antibody having an amino acid sequence at least a part of which is derived from an IgA antibody.

The dimeric IgA antibody used in the production method of the present disclosure preferably does not comprise SC. The dimeric IgA antibody used in the production method of the present disclosure comprises a J chain.

The dimeric IgA antibody and the monomeric IgA antibody to be mixed may be produced by a method known in the art. For example, the dimeric IgA antibody and the monomeric IgA antibody may be separately produced in separate cultured cells. The dimeric IgA antibody may be produced, for example, by co-expressing a heavy chain protein, a light chain protein and a J chain for constituting the IgA antibody in one host cell. The monomeric IgA antibody may be produced, for example, by co-expressing a heavy chain protein and a light chain protein for constituting the IgA antibody in one host cell.

Examples of the host cell include mammalian cells, insect cells, bacteria, and yeast. Examples of mammalian cells include, but not limited to, a 293F cell, and a CHO cell. Examples of insect cells include, but not limited to, cell line Sf9, and cell line Sf21.

A step of co-expressing the above-mentioned proteins (a heavy chain protein, a light chain protein and a J chain for the dimeric IgA antibody; a heavy chain protein and a light chain protein for the monomeric IgA antibody) in one host cell may be performed, for example, by introducing an expression vector(s) containing nucleic acids encoding these proteins into the cell. Such expression vectors and introduction methods of the vectors are well known in the art. Examples of the expression vectors include phage vectors, virus vectors, and plasmid vectors. Suitable vectors for the host are appropriately selected by those skilled in the art. After introduction of the expression vector, the cell is cultured to express the desired proteins. Cell culture conditions are appropriately selected by those skilled in the art.

In the production method of the present disclosure, in addition to the dimeric IgA antibody and the monomeric IgA antibody or the dimeric IgA antibody, the monomeric IgA antibody and SC, at least one substance selected from the group consisting of a molecular chaperone protein, a disulfide bond isomerase, oxidized glutathione, and reduced glutathione may be further mixed. The molecular chaperone protein is involved in folding of protein molecules. Examples of the molecular chaperone protein include Hsp60 family, Hsp70 family, Hsp90 family, Hsp100 family, low-molecular-weight Hsp family, isomerases, and cofactors thereof. Specific examples thereof include Dnak, DnaJ, GrpE, GroE, GroEL, and GroES. Two or more molecular chaperone proteins may be used. The disulfide bond isomerase is an enzyme involved in a disulfide bond between amino acid residues. The oxidized glutathione creates oxidative conditions necessary for formation of a disulfide bond. Use of at least one of these substances promotes formation of tetrameric IgA antibodies. In the production method of the present disclosure, preferably at least two substances selected from the group consisting of a molecular chaperone protein, a disulfide bond isomerase, oxidized glutathione, and reduced glutathione may be further mixed, and more preferably a molecular chaperone protein, a disulfide bond isomerase, oxidized glutathione and reduced glutathione are further mixed. In the production method of the present disclosure, preferably at least one substance selected from the group consisting of a molecular chaperone protein and a disulfide bond isomerase may be further mixed.

In the production method of the present disclosure, mixing of the dimeric IgA antibody and the monomeric IgA antibody, or mixing of the dimeric IgA antibody, the monomeric IgA antibody and SC, or mixing of the dimeric IgA antibody, the monomeric IgA antibody, and at least one substance selected from the group consisting of a molecular chaperone protein, a disulfide bond isomerase, oxidized glutathione and reduced glutathione, or mixing of the dimeric IgA antibody, the monomeric IgA antibody, SC, and at least one substance selected from the group consisting of a molecular chaperone protein, a disulfide bond isomerase, oxidized glutathione and reduced glutathione may be performed in a buffer adjusted to pH 6-10, preferably pH 7-8. Examples of the buffer include, but not limited to, a phosphate buffer, a Tris-HCl buffer, and a Good's buffer. Suitable temperature for mixing can be appropriately determined by those skilled in the art. Examples of the suitable temperature include 25°C to 45°C, preferably 30°C to 43°C, more preferably 35°C to 40°C. For example, the temperature may be around 37°C. A mixing time is not particularly limited, and can be appropriately determined by those skilled in the art based on other conditions such as a reaction volume. For example, the mixing may be performed for 6 hours or more, for 12 hours or more, for 24 hours or more, or for 48 hours or more, or preferably for a period of about 24 hours to 72 hours. A mixing ratio of the dimeric IgA antibody and the monomeric IgA antibody, or a mixing ratio of the dimeric IgA antibody, the monomeric IgA antibody and SC, or a mixing ratio of the dimeric IgA antibody, the monomeric IgA antibody, and at least one substance selected from the group consisting of a molecular chaperone protein, a disulfide bond isomerase, oxidized glutathione and reduced glutathione, or a mixing ratio of the dimeric IgA antibody, the monomeric IgA antibody, SC, and at least one substance selected from the group consisting of a molecular chaperone protein, a disulfide bond isomerase, oxidized glutathione and reduced glutathione is not particularly limited, and can be appropriately determined by those skilled in the art.

The trimeric and tetrameric IgA antibodies thus produced can be separated from other IgA antibodies (the dimeric IgA antibodies and the monomeric IgA antibodies that have not been polymerized) based on their properties such as molecular size. In addition, the trimeric Ig antibody and the tetrameric IgA antibody can also be separated from each other. For separation of the trimeric and tetrameric IgA antibodies from other IgA antibodies, for example, size exclusion chromatography, ultrafiltration, ion exchange chromatography, mass spectrometry, etc. can be used. Thus, the production method of the present disclosure may further comprise a step of separating the trimeric and tetrameric IgA antibodies from other IgA antibodies.

The specificity of the trimeric and tetrameric IgA antibodies produced by the production method of the present disclosure can be determined by a known method, for example ELISA, size exclusion chromatography, or affinity chromatography. Most of the polymeric IgA antibodies produced by the production method of the present disclosure are tetrameric IgA antibodies. Thus, according to the production method of the present disclosure, a mixture of trimeric IgA antibodies and tetrameric IgA antibodies comprising a higher proportion of tetrameric IgA antibodies than trimeric IgA antibodies is obtained.

### 3. Multispecific polymeric IgA antibody

The second aspect of the present invention provides a trimeric or tetrameric IgA antibody having at least bispecificity, comprising a first Fab region containing a first antigen binding site and a second Fab region containing a second antigen binding site (hereinafter also referred to as the "multispecific polymeric IgA antibody of the present disclosure"). As a further aspect of the multispecific polymeric IgA antibody of the present disclosure, provided is a trimeric or tetrameric IgA antibody having at least bispecificity, comprising a first Fab region containing a first antigen binding site, a second Fab region containing a second antigen binding site, and SC. The multispecific polymeric IgA antibody of the present disclosure is preferably obtained by the production method of the present disclosure as described above.

The multispecific polymeric IgA antibody is preferably a polymer composed of one dimeric IgA antibody and one or two monomeric IgA antibodies. The multispecific polymeric IgA antibody is more preferably a polymer composed of one dimeric IgA antibody, one or two monomeric IgA antibodies, and SC. For example, one dimeric IgA antibody and one or two monomeric IgA antibodies are trimerized or tetramerized via SC. For example, the dimeric IgA antibody comprises a first antigen binding site and the monomeric IgA antibody comprises a second antigen binding site. In a preferred example, the multispecific polymeric IgA antibody of the present disclosure comprises four first Fab regions and at least two second Fab regions. In a further preferred example, the multispecific polymeric IgA antibody of the present disclosure comprises four first Fab regions, at least two second Fab regions, and SC. In still further preferred examples, the multispecific polymeric IgA antibody of the present disclosure may be a bispecific trimeric IgA antibody comprising four first Fab regions and two second Fab regions, or a bispecific tetrameric IgA antibody comprising four first Fab regions and four second Fab regions, or a trispecific tetrameric IgA antibody comprising four first Fab regions, two second Fab regions, and two third Fab regions. In still further preferred examples, the multispecific polymeric IgA antibody of the present disclosure may be a bispecific trimeric IgA antibody comprising four first Fab regions, two second Fab regions and an SC, or a bispecific tetrameric IgA antibody comprising four first Fab regions, four second Fab regions and SC, or a trispecific tetrameric IgA antibody comprising four first Fab regions, two second Fab regions, two third Fab regions and SC. In a still further preferred example, said four first Fab regions are derived from the dimeric IgA antibody, and said two or four second or third Fab regions are derived from the monomeric IgA antibodies.

The multispecific polymeric IgA antibody of the present disclosure may be either IgA1 or IgA2, or may comprise both. Preferably, the multispecific polymeric IgA antibody of the present disclosure may comprise both IgA1 and IgA2. More preferably, the multispecific polymeric IgA antibody may comprise four Fab regions derived from dimeric IgA1 and two or four Fab regions derived from monomeric IgA2. The IgA2 comprised in the multispecific polymeric IgA antibody of the present disclosure may be IgA2m1, IgA2m2, or IgA2(n).

The multispecific polymeric IgA antibody of the present disclosure may comprise a hydrophobic amino acid residue at position 458 in its heavy chain constant region. Examples of the hydrophobic amino acid include isoleucine, leucine, methionine, tryptophan, and glycine, and preferably, isoleucine.

The multispecific polymeric IgA antibody of the present disclosure may be a recombinant IgA antibody. For example, when the multispecific polymeric IgA antibody of the present disclosure is a polymer composed of one dimeric IgA antibody and one or two monomeric IgA antibodies or a polymer composed of one dimeric IgA antibody, one or two monomeric IgA antibodies and SC, any or all of the dimeric IgA antibody and the monomeric IgA antibody(ies) may be recombinant IgA antibodies.

The IgA antibodies and SC for constituting the multispecific polymeric IgA antibody of the present disclosure are as above described in section "2. Production method for polymeric IgA antibody".

### 4. Pharmaceutical composition

The third aspect of the present invention provides a pharmaceutical composition comprising the multispecific polymeric IgA antibody of the present disclosure (hereinafter also referred to as the "pharmaceutical composition of the present disclosure"). The pharmaceutical composition of the present disclosure preferably comprises the multispecific polymeric IgA antibody of the present disclosure or a mixture of said multispecific polymeric IgA antibodies as an active ingredient.

The pharmaceutical composition of the present disclosure can be used, for example, for treatment, prevention or diagnosis of diseases. Examples of the diseases for which the pharmaceutical composition of the present disclosure is used include, but not limited to, infections, for example infections caused by pathogens such as parasites, bacteria, fungi, viruses, abnormal prions, etc., and malignant tumors. Further examples of infections include mucosal infections, such as influenza virus infection, respiratory syncytial virus infection, Ebola virus infection, severe fever with thrombocytopenia syndrome (SETS), severe acute respiratory syndrome (SARS), Middle East respiratory syndrome (MERS), acquired immunodeficiency syndrome (AIDS), etc.

The multispecific polymeric IgA antibody comprised in the pharmaceutical composition of the present disclosure has suitable antigen binding sites for the intended use of the pharmaceutical composition. For example, when used for diagnosis, prevention, treatment, etc. of a disease, the multispecific polymeric IgA that specifically binds to two different proteins related to a target disease (e.g., causative protein, marker protein, etc.) can be used. For example, the multispecific polymeric IgA antibody comprised in the pharmaceutical composition of the present disclosure may specifically bind to both HA and NA proteins of influenza virus. For example, the multispecific polymeric IgA antibody comprised in the pharmaceutical composition of the present disclosure may specifically bind to both a target cell-specific protein and a therapeutically effective protein. Examples of antigens or epitopes that can be targeted by the multispecific polymeric IgA antibody comprised in the pharmaceutical compositions of the present disclosure include, but not limited to, two or more epitopes of Ebola virus glycoprotein, Gn protein and Gc protein that are SETS virus glycoprotein, lymphocyte marker proteins such as CD3 and CD8, and cancer-specific antigens.

The pharmaceutical composition of the present disclosure can be formulated and administered in a dosage form such as powder, liquid, etc. The pharmaceutical composition of the present disclosure may further contain an excipient, an additive, a thickener and the like known in the pharmaceutical field. For example, the pharmaceutical composition of the present disclosure may be administered by spraying onto a nasal mucosa, by inhalation into a lower respiratory tract using a nebulizer, or the like.

The pharmaceutical composition of the present disclosure may be intended for humans, or non-human mammals including domestic animals such as horse, cow, goat, sheep, pig etc., pet animals such as dog, cat etc., primates such as chimpanzee, gorilla, cynomolgus etc., and rodents such as mouse, rat, guinea pig etc.

Any of the heavy chain, light chain, SC and J chain of the multispecific polymeric IgA antibody comprised in the pharmaceutical composition of the present disclosure preferably comprises an amino acid sequence derived from an animal that is a subject for administration of the pharmaceutical composition (the subject animal-type amino acid sequence). More preferably, all of the heavy chain, light chain, SC and J chain of the multispecific polymeric IgA antibody comprised in the pharmaceutical composition of the present disclosure comprise amino acid sequences derived from an animal that is a subject for administration of the pharmaceutical composition (the subject animal-type amino acid sequences). As used herein, with respect to the heavy chain and light chain, the term "subject animal-type" means that the constant regions of the heavy chain and light chain of the multispecific polymeric IgA antibody have amino acid sequences of the constant regions of an IgA heavy chain and an IgA light chain derived from the animal that is a subject for administration of the pharmaceutical composition. The amino acid sequences of the IgA heavy chain, IgA light chain, SC and J chain may comprise a mutation (s) as long as they have the desired antigen-binding activity.

### Examples

Hereinafter, the present invention is further explained using Examples to which the present invention is not limited.

### [Materials and Preparation]

### 1. Preparation of IgA expression plasmid vector

An expression vector for an α1 heavy chain (HC) human IgA1 constant region was prepared as follows. PCR was performed using PrimeSTARTM (registered trademark) MAX DNA Polymerase (Takara Bio, Kusatsu, Japan). Briefly, a human IgA1 antibody constant region gene was amplified by PCR using pFUSE-CHIg-hA1 (InvivoGen) as a template and an appropriate primer set for amplifying the IgA1 antibody constant region containing sites recognized by restriction enzymes XhoI and HindlII. The PCR were performed for 30 cycles, each consisting of 98°C for 10 seconds, 55°C for 5 seconds, and 72°C for 10 seconds. Subsequently, PCR was performed using a human γ1HC expression vector as reported by T. Tiller et al. (J Immunol Methods, 329, 112-24, 2008) as a template and an appropriate primer set for removing a γ1HC human IgG1 antibody constant region containing sites recognized by XhoI and HindIII and excluding a signal sequence. The PCR were performed for 30 cycles, each consisting of 98°C for 10 seconds, 55°C for 5 seconds, and 72°C for 30 seconds. PCR products were purified by using MonoFas DNA Purification Kit I (GL Sciences Inc., Tokyo, Japan). The purified α1HC and the purified γ1HC-removed expression vector of T. Tiller et al. were treated with restriction enzymes XhoI (New England Biolabs) and HindIII-HF (New England Biolabs) at 37°C. Restriction enzyme-treated products were purified using MonoFas DNA Purification Kit I. The restriction enzyme-treated DNAs were ligated using DNA Ligation Kit <Mighty Mix> (Thermo Fisher Scientific) . A total amount of a ligation product was transformed into Competent Quick DH5α at 42°C. Plasmid extraction was performed by PureYield^{™} Plasmid Miniprep System (Promega). The extracted plasmid was sequenced using an Applied Biosystems 3130 Genetic Analyzer. A sequencing reaction was performed using a BigDye Terminator v3.1 Cycle Sequencing kit. Purification was performed using a BigDye XTerminator^{™} kit. All the above-described operations were carried out according to attached instructions.

Human IgA2 allotype α2m2 HC was artificially synthesized (GeneArt Strings DNA Fragments) based on human codon-optimized sequences of sequences registered on IMGT/GENE-DB (accession numbers for α2m2 HC: M60192 and AJ012264). To facilitate cloning of variable regions, codons for the last amino acid, alanine, of the variable region and the first amino acid, serine, of the constant region were changed to create an NheI cleavage site. The synthetic sequence treated with NheI-HF (New England Biolabs) and HindIII-HF and the α1 HC treated with the same restriction enzymes were purified using MonoFas DNA purification kit I. Ligation of the restriction enzyme-treated DNAs was performed using DNA Ligation Kit <Mighty Mix> (Thermo Fisher Scientific) . A portion of a ligation product was transformed into Competent Quick DH5α at 42°C. Plasmid extraction was performed by PureYield^{™} Plasmid Miniprep System (Promega). The extracted plasmid was sequenced using an Applied Biosystems 3130 Genetic Analyzer. A sequencing reaction was performed using a BigDye Terminator v3.1 Cycle Sequencing Kit. Purification was performed using a BigDye XTerminator^{™} Kit. All the above-described operations were carried out according to attached instructions.

Since a human light chain (LC) is common among antibody isotypes, a γ LC expression vector as reported by T. Tiller et al. was used.

### 2. Cloning of antibody variable region gene into α1, α2m2 HC, and λ LC expression vectors

F045-092, known as an antibody clone that broadly binds to hemagglutinin (HA) of influenza virus (H3N2), was used. For F045-092, sequences registered on Nucleotide (accession numbers: AB649270 for heavy chain, AB649271 for light chain) were codon-optimized for human, adjusted to be incorporated into the α1 HC (heavy chain), α2m2 HC (heavy chain), and λ LC (λ chain) expression vectors, and then artificially synthesized (GeneArt Strings DNA Fragments). The synthetic sequences treated with AgeI-HF (all chains), NheI-HF (heavy chain), and XhoI (λ chain) (New England Biolabs), and the α1 HC, α2m2 HC, and XLC expression vectors treated with the same restriction enzymes were purified using MonoFas DNA Purification Kit I. Ligation of the restriction enzyme-treated DNAs was performed using DNA Ligation Kit <Mighty Mix> (Thermo Fisher Scientific). A portion of a ligation product was transformed into Competent Quick DH5α at 42°C. Plasmid extraction was performed by PureYield^{™} Plasmid Miniprep System (Promega). The extracted plasmid was sequenced using an Applied Biosystems 3130 Genetic Analyzer. A sequencing reaction was performed using a BigDye Terminator v3.1 Cycle Sequencing Kit. Purification was performed using a BigDye XTerminator^{™} Kit. All the above-described operations were carried out according to attached instructions.

### 3. Preparation of expression plasmid DNA for fluorescence-labeled IgA1 (mC-A1) and IgAm2 (mC-A2) antibodies

IgA1Δ and IgA2m2Δ mutants composed of CH2 and CH3 and lacking up to the 241st cysteine in heavy chains of respectively α1 and α2m2, which are different subclasses of IgA antibodies, were produced by PCR using a specific primer set to which a restriction enzyme site was added and PrimeSTAR Max DNA Polymerase (Takara Bio, Kusatsu, Japan). In addition, a sequence of fluorescent protein mCherry was specifically amplified by performing PCR using a primer set with recognition sequences for restriction enzyme AgeI added to the 5' and 3' ends, and PrimeSTAR Max DNA Polymerase (Takara Bio). The IgAΔ mutants and mCherry were treated with restriction enzyme AgeI (New England Biolabs) under optimal conditions, and then subjected to agarose gel electrophoresis to obtain only bands of desired products that were digested by the restriction enzyme. The desired products thus obtained were purified using MonoFas DNA Purification Kit I (GL Sciences Inc., Tokyo, Japan). The purified DNA fragment encoding each IgAΔ mutant and the purified DNA fragment encoding mCherry were mixed at a ratio of 1:3 (= IgA1Δ : mCherry), and ligated using an equal amount of DNA Ligation Kit <Mighty Mix> (Thermo Fisher Scientific) at 16°C for 30 minutes. Cyclized IgAΔ and mCherry were transformed into E. coli DH5α Competent Cells (Takara Bio, Kusatsu, Japan) at 42°C. Plasmid DNA extraction was performed by PureYield Plasmid Miniprep System (Promega). Sequence analysis was performed using Sanger method. The mCherry was added at the 5' side of the sequences encoding IgA1Δ and IgA2m2Δ. The IgAΔ mutants fused with fluorescent protein mCherry were named an mC-A1 antibody and an mC-A2 antibody, respectively. All the operations performed were in accordance with attached instructions.

### 4. Cloning of J chain and secretory component (SC)

A J chain (GenBank accession no. NM_144646) in which a recognition sequence for XhoI and a Kozak sequence were added to the 5' side of a coding sequence and a recognition sequence for NotI was added to the 3' side of the coding sequence was artificially synthesized by Artificial Gene Synthesis Service (Eurofins Genomics). The J chain was treated with XhoI (New England Biolabs) and NotI-HF (New England Biolabs) under optimal conditions. Under the same conditions, a pCXSN vector, which is an expression vector for mammalian cultured cells, was treated with the restriction enzymes. Ligation of the restriction enzyme-treated DNAs was performed using DNA Ligation Kit <Mighty Mix> (Thermo Fisher Scientific). A portion of a ligation product was transformed into Competent Quick DH5α at 42°C. Plasmid extraction was performed by PureYield^{™} Plasmid Miniprep System (Promega). The extracted plasmid was sequenced using an Applied Biosystems 3130 Genetic Analyzer. A sequencing reaction was performed using a BigDye Terminator v3.1 Cycle Sequencing Kit. Purification was performed using a BigDye XTerminator^{™} Kit. All the above-described operations were carried out according to attached instructions.

For a wild-type SC (SC-wt), a DNA fragment (SEQ ID NO: 7) comprising 1809 bp (comprising a signal sequence) from the 5' end of pIgR (GenBank accession no. NM_002644) and containing an XhoI recognition sequence and a kozak sequence at the 5' side and a HindIII recognition sequence, a Thrombin cleavage sequence and a 6x His tag at the 3' side was artificially synthesized using GeneArt (registered trademark) Strings DNA Fragments (Life Technologies). The synthesized DNA fragment was subjected to PCR using PrimeSTAR Max DNA Polymerase (Takara Bio, Kusatsu, Japan) and DNA purification. The DNA fragment was then treated with restriction enzymes XhoI and HindIII under optimal conditions. Under the same conditions, a pCXSN vector, which is an expression vector for mammalian cultured cells, was treated with the restriction enzymes. Ligation of the restriction enzyme-treated DNAs was performed using DNA Ligation Kit <Mighty Mix> (Thermo Fisher Scientific). A portion of a ligation product was transformed into Competent Quick DH5α at 42°C. Plasmid extraction was performed by PureYield^{™} Plasmid Miniprep System (Promega). The extracted plasmid was sequenced using an Applied Biosystems 3130 Genetic Analyzer. A sequencing reaction was performed using a BigDye Terminator v3.1 Cycle Sequencing Kit. Purification was performed using a BigDye XTerminator^{™} Kit. All the above-described operations were carried out according to attached instructions. The gene sequence encoding SC-wt is shown in Figure 2-2.

### 5. Preparation of expression plasmid DNA for SC12 deletion mutant

A sequence encoding domain 1 and domain 2 of SC was amplified by performing PCR using an SC comprising five domains as a template, specific primers and PrimeSTAR Max DNA Polymerase (Takara Bio, Kusatsu, Japan), under optimal conditions in consideration of the optimal primer set and nucleotide length. Each domain of SC was determined according to a previous study by Beth et al. (eLife, 5, e10640, 2016). After the PCR reaction, a PCR product was subjected to agarose gel electrophoresis to confirm that a desired base length was specifically amplified. The PCR product was purified using MonoFas DNA purification kit I (GL Sciences Inc., Tokyo, Japan). The purified DNA fragment was phosphorylated using T4 polynucleotide kinase (Takara Bio, Kusatsu, Japan), and circularly self-ligated using T4 DNA ligase (Takara Bio, Kusatsu, Japan). After completion of the ligation procedure, a plasmid DNA (SEQ ID NO: 8) encoding the circularized deletion mutant was transformed into E. coli. DH5α Competent Cells (Takara Bio, Kusatsu, Japan) at 42°C. Plasmid DNA extraction was performed by PureYield Plasmid Miniprep System. The extracted plasmid was sequenced using an Applied Biosystems 3130 Genetic Analyzer. A sequencing reaction was performed using a BigDye Terminator v3.1 Cycle Sequencing Kit. Purification was performed using a BigDye XTerminator^{™} Kit. All the above-described operations were carried out according to attached instructions. All the operations performed were in accordance with attached instructions. The gene sequence encoding the SC12 deletion mutant is shown in Figures 2-3.

### 6. Expression of monomeric IgA antibody and dimeric IgA antibody

The components of a monomeric IgA antibody are a heavy chain and a light chain. A dimeric IgA antibody is composed of a heavy chain, a light chain and a J chain. The plasmid DNAs for expressing molecules constituting monomeric and dimeric IgA antibodies were co-introduced into 2.9 × 10⁶ cells/mL of Expi293F human cell (Thermo Fisher Scientific), which is a mammalian cultured cell derived from a human kidney cell, using Expi293 Expression System Kit (Thermo Fisher Scientific, Waltham, Massachusetts, USA). The cells were then cultured with shaking at 37°C, 8% CO₂, and 120 rpm. A week after, the cell culture medium was collected.

### 7. Expression of mC-A1 and mC-A2 antibodies

Since the mC-A1 and mC-A2 antibodies did not have light chains, the plasmid DNAs encoding each heavy chain and the J chain were co-introduced into 2.9 × 10⁶ cells/mL of Expi293F human cell (Thermo Fisher Scientific), which is a mammalian cultured cell derived from a human kidney cell, using Expi293 Expression System Kit (Thermo Fisher Scientific, Waltham, Massachusetts, USA). The cells were then cultured with shaking at 37°C, 8% CO₂, and 120 rpm. A week after, the cell culture medium was collected.

### 8. Purification of recombinant IgA antibody and mC-A1/2

The collected cell culture medium was centrifuged at 3000 rpm for 20 minutes to remove debris such as cells, and only a culture supernatant was collected. This centrifugation operation was performed twice in total. The supernatant was then filtered using a glass fiber filter and Stericup (Merck KGaA, Darmstadt, Germany). The recombinant IgA antibodies and mC-A1/2 were purified using CaptureSelect IgA Affinity Matrix (Thermo Fisher Scientific), which specifically recognizes the constant regions of human IgA antibodies. A purification method was performed according to the manufacturer's instructions. A brief purification method is described below. A column was equilibrated with 10 CV of PBS and the filtered culture supernatant was loaded onto the column. The column was washed with 10 CV of PBS. The antibody was eluted with 5 CV of 0.1 M Glycine-HCl (pH 3.0). The eluate was neutralized with 1 M Tris-HCl (pH 8.0). The antibody was concentrated using Amicon (registered trademark) Ultra Centrifugal Filter Devices (Millipore) according to the manufacturer's instructions.

### 9. Separation of monomeric and dimeric IgA antibodies by gel filtration chromatography

After concentration of the monomeric and dimeric IgA antibodies, they were fractionated by gel filtration chromatography. For fractionation of the recombinant IgA antibodies, Superose 6 10/300 GL (GE Healthcare) was used. For fractionation of mC-A1/2, Superose 12 10/300 GL was used. The gel filtration chromatography was performed according to the manufacturer's instructions. The chromatography was performed using 1.5 CV of PBS for column equilibration and 0.2 mL/fraction (total 1.5 CV) of PBS for elution, at a flow rate of 0.5 mL/min. Fractions containing monomeric and dimeric IgA antibodies were concentrated using Amicon (registered trademark) Ultra Centrifugal Filter Devices. The concentration was measured with NanoDrop.

### 10. Expression and purification of recombinant SC12

The plasmid DNA encoding SC12 was introduced into 2.9 × 10⁶ cells/mL of Expi293F human cell (Thermo Fisher Scientific), which is a mammalian cultured cell derived from a human kidney cell, using Expi293 Expression System Kit (Thermo Fisher Scientific, Waltham, Massachusetts, USA). The cells were then cultured with shaking at 37°C, 8% CO₂, and 120 rpm. A week after, the cell culture medium was collected. The collected cell culture medium was centrifuged at 3000 rpm for 20 minutes to remove debris such as cells, and only a culture supernatant was collected. This centrifugation operation was performed twice in total. SC12 in the culture supernatant was purified using an affinity column packed with Ni Sepharose excel (GE Healthcare) that recognizes a C-terminal His tag of each protein. Briefly, an equilibration solution containing 20 mM sodium phosphate, 0.5 M NaCl, pH 7.4, a washing solution containing 20 mM sodium phosphate, 0.5 M NaCl, 10 mM imidazole, pH 7.4, and an eluting solution containing 20 mM sodium phosphate, 0.5 M NaCl, 500 mM imidazole, pH 7.4 were used. After a column was equilibrated with 5 CV (column volume) of the equilibration solution, the culture supernatant was passed through the column. The column was washed with 20 CV of the washing solution, eluted with 5 CV of the eluting solution, and re-equilibrated with 5 CV of the equilibration solution. SC12 was concentrated using Amicon (registered trademark) Ultra Centrifugal Filter Devices (Millipore) according to the manufacturer's instructions.

### [Example 1: Formation of polymeric IgA antibody outside cell]

Our previous study (Patent Literature 1) found that a heavy chain (HC), a light chain (LC), a J chain (JC) and a secretory component (SC) for constituting trimeric and tetrameric secretory IgA antibodies (tSIgA) were co-expressed in a cultured mammalian cell to produce efficiently tSIgA. Since the formation efficiency of tSIgA was varied depending on the presence or absence of SC, it was suggested that SC could function as a factor for promoting the formation of trimeric and tetrameric IgA antibodies in cultured cells. This ability of SC to promote the formation of trimeric and tetrameric IgA antibodies was evaluated by measuring trimeric and tetrameric IgA antibodies (Tri/Tet IgA) formed by interaction of the four types of proteins coexisting in the cell. This Example evaluated whether SC functioned as a factor for promoting the formation of polymeric IgA antibodies from isolated proteins that were produced from separate cells, in extracellular conditions instead of a confined environment within cells. SC12 composed of SC domains 1 and 2 was shown to have a higher ability to promote the trimer- and tetramer-formation within cells than SC-wt. Thus SC12 was used in this Example.

The SC12, monomeric IgA1 antibody (mA1) and dimeric IgA1 antibody (dA1) prepared as described above were mixed in a phosphate buffer (pH 7.4) at each final concentration of 1.25 mg/mL, and reacted at 37°C for a week using a rotator. Then, the antibody sample was pretreated using a Cosmospin filter H (Nacalai Techs). Tri/Tet IgA formed in the reaction solution was separated based on molecular size by size exclusion chromatography (SEC), and then detected by UV (280 nm) to evaluatea degree of antibody polymerization. As an HPLC system, Agilent 1260 Infinity (Agilent Technologies) was used. As a column, KW404-4F (Shodex) was used. A phosphate buffer pH 7.4 was used as an eluting solution at a flow rate of 0.2 mL/min. Nine µL of each IgA antibody mixed solution was used. Chromatograms were analyzed using OpenLAB CDS ChemStation Edition (Agilent Technologies).

Results are shown in FIG. 3. In the figure, "mA1+dA1+SC12" shows a result when the monomeric IgA antibody, the dimeric IgA antibody and SC12 were mixed. "mA1+SC12" shows a result when the monomeric IgA antibody and SC12 were mixed. "dA1+SC12" shows a result when the dimeric IgA antibody and SC12 were mixed. As controls, trimeric and tetrameric IgA antibodies (Tri/Tet IgA) produced intracellularly by using the method described in Patent Literature 1 were used.

As a result, no peak of the trimeric/tetrameric IgA antibodies was detected when the combination of "mA1+SC12" or the combination of "dA1+SC12" was used. On the other hand, when the three types of proteins "mA1+dA1+SC12" were mixed, a peak of polymeric IgA antibody having a larger molecular weight than dA1 was detected at a detection time equivalent to that of the intracellularly produced "Tri/Tet IgA". These results showed that coexistence of mA1 and dA1 in addition to SC12 was required for the formation of trimeric/tetrameric IgA antibodies. Furthermore, it was found that the trimeric/tetrameric IgA antibodies can be formed outside cells, in the same way as within cells. Furthermore, it was shown that polymerization between monomers or dimers did not occur. Therefore, it was found that the trimeric/tetrameric IgA antibodies were formed by polymerization of the dimeric IgA antibody and the monomeric IgA antibody.

### [Example 2: Comparison of polymeric IgA antibody formation in the presence of SC12 and SC-wt]

Since Example 1 demonstrated that polymeric IgA antibodies were formed extracellularly by mixing SC and monomeric and dimeric IgA antibodies. Thus it was found that SC is involved in the formation of polymeric IgA antibodies. Our previous study found that SC12 had higher ability to promote the trimer/tetramer formation under intracellular conditions than SC-wt. Thus, in order to search for a method for efficiently producing trimeric/tetrameric IgA antibodies under extracellular conditions, in this Example, the ability to promote the tetramer formation was compared between SC12 and SC-wt.

The SC12, SC-wt, mA1 and dA1, and mCherry-fused IgA antibody (mC-A1) monomer (mC-mA1) and dimer (mC-dA1) in which fluorescence protein mCherry was substituted for a region corresponding to Fab of an IgA antibody, prepared as described above were mixed in a phosphate buffer (pH 7.4) at each final concentration of 0.5 mg/mL, and reacted at 37°C for 6 hours using a rotator. The trimeric/tetrameric IgA produced in the reaction solution was detected by an ELISA method.

The ELISA method was performed as follows. After 50 µl of a recombinant HA [derived from A/Sydney/5/1997 (H3N2), 5 µg HA/mL] was immobilized in a 96-well half plate at 4°C overnight, 1% BSA-PBS was added to the plate. The plate was incubated at room temperature for 1 hour for blocking. Then, a 3-fold serial dilution series of a 200-fold diluted antibody sample was prepared, added to each well, and reacted at 37°C for 2 hours. After washing with PBST, the antibody captured on the plate via the HA antigen was reacted with 5 ng/mL of Direct-Blot HRP anti-mCherry (BioLegend) at 37°C for 1 hour. After washing with PBST, the plate was subjected to chromogenic reaction with Immobilon Western Chemilum HRP Substrate (MERCK), and an absorbance at a wavelength of 450 nm was measured. In analysis, a coefficient was calculated for each plate so that the OD value of a control was 1.5, and correction between the plates was carried out by multiplying a measured value for each sample by the coefficient.

Results are shown in FIG. 4. When mA1 and mC-dA1 were reacted in the presence of SC12 or SC-wt, the OD value was higher than when mC-mA1 and dA1 were reacted. In comparison between the effects of SC12 and SC-wt, there was no difference when mC-mA1 was used, whereas a higher OD value was showed in the presence of SC12 when mC-dA1 was used. From these results, it was found that SC12 had a high ability to promote the formation of polymeric IgA antibodies. The IgA antibodies used in this Example were IgA antibodies having Fab regions comprising the variable regions of F045-092, and mC-A1. These antibodies had the same constant regions and different variable regions. Thus it was shown that the production method for polymeric IgA antibodies of the present disclosure is independent from variable regions, and can produce IgA antibodies having two or more different specificities, preferably bi- or trispecific IgA antibodies. Furthermore, it was shown that polymeric IgA antibodies can be formed even when a dimeric IgA antibody and a monomeric IgA antibody having different variable regions are mixed.

### [Example 3: Identification of factor relating to promotion of polymer formation - 1]

Example 2 demonstrated that SC12 had a higher ability to promote the formation of polymeric IgA antibodies than SC-wt. Thus, in order to further enhance the formation of polymeric antibodies, addition of protein components other than SC and IgA antibodies was tested.

The SC12, SC-wt, mA1 and dA1 as well as mC-mA1 and mC-dA1 prepared as described above at each final concentration of 0.5 mg/mL, and an additive (Supple) were mixed in a phosphate buffer (pH 7.4), and reacted at 37°C for 6 hours using a rotator. Supple contained chaperone proteins DnaK Mix (5 µM DnaK, 1 µM DnaJ, 1 µM GrpE) and GroE Mix (0.5 µM GroEL, 1 µM GroES), disulfide bond isomerase DsbC (375 µg/mL), and oxidized glutathione GSSG (30 mM). Trimeric/tetrameric IgA produced in the reaction mixture was detected by the ELISA method in the same manner as in Example 2.

Results are shown in FIG. 5A and FIG. 5B. In the presence of SC12, addition of Supple resulted in an increase of an OD value that was indicative of trimeric/tetrameric IgA formation in both mixtures of "mA1 + mC-dA1" and "mC-mA1 + dA1" (FIG. 5A). The OD value was increased by two or more times in both mixtures. Similarly, in the presence of SC-wt, addition of Supple significantly increased an OD value to the same level as that when SC12 was added (FIG. 5B).

### [Example 4: Identification of factor relating to promotion of polymer formation - 2]

A contribution of each factor to the polymer formation was evaluated by reacting mC-mA1 and dA1 with each of the four major components contained in Supple in the presence of SC12. Reaction and detection were performed in the same manner as in Example 3, except that DnaK Mix, GroE Mix, GSSG, or DsbC was added alone as an additive.

Results are shown in FIG. 6. When DnaK Mix or GroE Mix, which act as molecular chaperones, were added, similar OD values were observed. On the other hand, when disulfide bond isomerase DsbC was added, the highest OD value was observed among the four components. However, when oxidized glutathione GSSG, which creates oxidative conditions necessary for disulfide bond formation, was added, the lowest efficiency of promoting the trimer/tetramer IgA formation was shown among the four components. These results showed that Supple used in this Example was useful for promoting trimerization/tetramerization of IgA antibodies.

### [Example 5: Identification of factor relating to promotion of polymer formation - 3]

This Example was focused on the constant region of IgA antibody as a factor involved in the polymerization of IgA antibody. IgA antibodies are classified into two subclasses: IgA1 and IgA2, depending on the constant regions. Further, IgA2 is classified into three allotypes: IgA2m1, IgA2m2, and IgA2(n). Our previous study (Patent Literature 1) found that differences in the constant regions resulted in different efficiency of trimeric/tetrameric IgA antibody formation under intracellular conditions. Thus, in this Example, it was evaluated whether differences in the efficiency of polymer formation were observed due to different IgA antibody subclasses under extracellular conditions. Specifically, the efficiency of polymer formation due to different constant regions of IgA antibodies was compared by using different subclasses, IgA1 and IgA2m2.

In the same manner as in Example 3, SC12 was added to combinations of the mC-A1 and mC-A2 as well as the monomeric and dimeric IgA antibodies prepared as described above, and Supple was further added thereto. After reaction, the formation of trimeric/tetrameric IgA was evaluated.

Results are shown in FIG. 7. When the IgA2m2 antibody and the IgA1 antibody were combined, an enhanced OD value was observed as compared to when the monomeric IgA1 antibody and the dimeric IgA1 antibody were combined. These results showed that IgA2m2 had a high tetramer-forming ability.

### [Example 6: Identification of factor relating to promotion of polymer formation - 4]

As a factor affecting the polymerization of IgA antibodies, reaction time was investigated. In the presence of SC12 and Supple, mC-mA1 and dA1 were reacted in a phosphate buffer (pH 7.4) at 37°C. Samples were collected 6, 12 and 24 hours after initiation of the reaction, and subjected to evaluation of trimeric/tetrameric IgA formation by ELISA.

Results are shown in FIG. 8. Though no significant change was observed, the OD value was enhanced by extending the reaction time. From these results, it was expected that the efficiency of polymeric IgA antibody formation would be further increased by using the optimal reaction time.

### [Example 7: Evaluation of bispecific IgA antibody using different subtypes of HA]

Example 2 demonstrated that trimeric/tetrameric IgA antibodies comprising different variable regions were formed by mixing the mCherry-fused monomeric and dimeric IgA antibodies in the presence of SC-wt and SC12. Furthermore, Example 5 demonstrated differences in the polymer-forming efficiency due to different IgA antibody subclasses. Thus, in this Example, trimeric/tetrameric IgA antibodies were formed by the method of the present disclosure using two different subtypes of influenza virus protein HA to which detection tags are added and IgA antibody clones having specificity for each HA, and the bispecificity of the obtained trimeric/tetrameric IgA antibodies was verified.

As the antibody clones, 18-18K (H1) and 15-19L (H3) having specificity for influenza A subtypes H1 and H3 respectively were used. These clones were isolated from subjects who participated in clinical trials of nasal influenza vaccines. Variable regions from these antibodies were cloned into the α1 HC, α2m2 HC, and λ LC expression vectors by the same methods as described above to produce a dimeric IgA1 antibody of antibody clone 18-18K having specificity for influenza virus A/California/7 /2009 (H1N1) (H1/HA), and a monomeric IgA2m2 antibody and a dimeric IgA1 antibody of antibody clone 15-19L having specificity for A/New York/39/2012(H3N2)(H3/HA). A nucleotide sequence and an amino acid sequence of the heavy chain variable region of 18-18K(H1) are shown in SEQ ID NO: 9 and SEQ ID NO: 10, respectively. A nucleotide sequence and an amino acid sequence of the light chain variable region of 18-18K(H1) are shown in SEQ ID NO: 11 and SEQ ID NO: 12, respectively. A nucleotide sequence and an amino acid sequence of the heavy chain variable region of 15-19L(H3) are shown in SEQ ID NO: 13 and SEQ ID NO: 14, respectively. A nucleotide sequence and an amino acid sequence of the light chain variable region of 15-19L(H3) are shown in SEQ ID NO: 15 and SEQ ID NO: 16, respectively. SC and SC12 were also prepared as described above.

The dimeric IgA1 antibody of 18-18K, the monomeric IgA2m2 antibody and the dimeric IgA1 antibody of 15-19L, and SC or SC12 were mixed in a phosphate buffer (pH 7.4) at each final concentration of 0.3 mg/mL. Specifically, SC-wt or SC12 was added to a mixture of the dimeric IgA1 antibody of 18-18K and the monomeric IgA2m2 antibody of 15-19L (Hetero group) and a mixture of the monomeric IgA2m2 antibody and the dimeric IgA1 antibody of 15-19L (Homo group), or neither SC-wt nor SC12 was added to Homo group and Hetero group. In addition, reduced glutathione (Fujifilm Wako Pure Chemical Industries, Ltd.) was added at a final concentration of 0.03 mM to Homo group and Hetero group, and reacted at 37°C for 12 hours using a thermal cycler. Trimeric/tetrameric IgA produced in the reaction was detected by an ELISA method.

The ELISA method was performed as follows. After 40 µl of strep-tagged recombinant H3/HA-Strept (derived from A/NewYork/39/2012(H3N2), 5 µg HA/mL) was immobilized in a 384-well plate at 4°C overnight, 1% BSA-PBS was added to the plate. The plate was incubated at room temperature for 1 hour for blocking. A 2-fold serial dilution series of a 100-fold diluted antibody sample was prepared, added to each well, and reacted at 37°C for 2 hours. After washing with PBST, 40 µl of His-tagged recombinant H1/HA-His (derived from A/California/7/2009(H1N1), 1 µg HA/mL) was added to each well and reacted at 37°C for 1 hour. After washing with PBST, 40 µl of 6000-fold diluted mAb-HRP-DirectT (MBL) was added to each well and reacted at 37°C for 1 hour. After washing with PBST, the plate was subjected to chromogenic reaction with Immobilon Western Chemilum HRP Substrate (MERCK), and an absorbance at a wavelength of 450 nm was measured.

Results are shown in FIG. 9. In Hetero group in which antibody clones having different specificities were combined, enhanced OD values were observed in the presence of SC as compared to the OD value in the absence of SC. Furthermore, similarly to the results of other Examples, a further enhanced OD value was observed in the presence of SC12, as compared to SC-wt. On the other hand, in Homo group in which the monomeric IgA2m2 (mA2) and dimeric IgA1 (dA1) having the same specificity were combined, OD values detected even in the presence of SC-wt or SC12 were not comparable to those detected in the absence of SC in Hetero group.

From these results, it was found that acquisition of the ability to bind to H1/HA was not due to the trimeric/tetrameric IgA antibody formation per se, but due to the fact that two types of IgA antibody molecules which were dA1 of antibody clone 15-19L having specificity for H3/HA and mA2 of antibody clone 18-18K having specificity for H1/HA formed a trimeric/tetrameric IgA antibody molecule. Even when IgA allotypes of IgA1 and IgA2m2 were combined in the absence of SC, trimeric/tetrameric IgA antibodies were formed. Furthermore, it was shown that trimeric/tetrameric IgA antibodies having bispecificity were produced more efficiently by reacting IgA antibodies in the presence of SC-wt or SC12.

### Sequence Listing Free Text

SEQ ID NO:1; Amino acid sequence of IgA1 constant region
SEQ ID NO:2; Amino acid sequence of IgA2m2 constant region
SEQ ID NO:3; Amino acid sequence of wild-type SC
SEQ ID NO:4; Amino acid sequence of SC12
SEQ ID NO:5; Amino acid sequence of wild-type SC containing signal sequence, thrombin site, His tag
SEQ ID NO:6; Amino acid sequence of SC12 containing signal sequence, thrombin site, His tag
SEQ ID NO:7; Nucleotide sequence of wild-type SC containing signal sequence, thrombin site, His tag
SEQ ID NO:8; Nucleotide sequence of SC12 containing signal sequence, thrombin site, His tag
SEQ ID NO:9; Nucleotide sequence of heavy chain variable region of 18-18K(H1 specific)
SEQ ID NO:10; Amino acid sequence of heavy chain variable region of 18-18K(H1 specific)
SEQ ID NO:11; Nucleotide sequence of light chain variable region of 18-18K(H1 specific)
SEQ ID NO:12; Amino acid sequence of light chain variable region of 18-18K(H1 specific)
SEQ ID NO:13; Nucleotide sequence of heavy chain variable region of 15-19L(H3 specific)
SEQ ID NO:14; Amino acid sequence of heavy chain variable region of 15-19L(H3 specific)
SEQ ID NO:15; Nucleotide sequence of light chain variable region of 15-19L(H3 specific)
SEQ ID NO:16; Amino acid sequence of light chain variable region of 15-19L(H3 specific)

## Claims

1. A method for producing trimeric and tetrameric IgA antibodies, the method comprising mixing a dimeric IgA antibody and a monomeric IgA antibody.

2. The method according to claim 1, which comprises further mixing a secretory component.

3. The method according to claim 1 or 2, wherein the dimeric IgA antibody contains a first antigen binding site and the monomeric IgA antibody contains a second antigen binding site, and the trimeric and tetrameric IgA antibodies have at least bispecificity.

4. The method according to claim 3, which comprises further mixing another monomeric IgA antibody containing a third antigen binding site.

5. The method according to claim 3, wherein each of four Fab regions of the dimeric IgA antibody contains the first antigen binding site, and each of two Fab regions of the monomeric IgA antibody containing the second antigen binding site contains the second antigen binding site.

6. The method according to claim 4, wherein each of four Fab regions of the dimeric IgA antibody contains the first antigen binding site, each of two Fab regions of the monomeric IgA antibody containing the second antigen binding site contains the second antigen binding site, and each of two Fab regions of the another monomeric IgA antibody contains the third antigen binding site.

7. The method according to any one of claims 2 to 6, wherein the secretory component is a wild-type SC or a mutant SC.

8. The method according to any one of claims 1 to 7, which comprises further mixing at least one substance selected from the group consisting of a molecular chaperone protein, a disulfide bond isomerase, oxidized glutathione, and reduced glutathione.

9. The method according to any one of claims 1 to 8, wherein the dimeric IgA antibody and the monomeric IgA antibody are recombinant IgA antibodies separately produced in cultured cells.

10. The method according to any one of claims 1 to 9, which further comprises separating the produced trimeric and tetrameric IgA antibodies from other IgA antibodies.

11. The method according to claim 10, which further comprises separating the produced trimeric IgA antibody from the produced tetrameric IgA antibody.

12. A trimeric or tetrameric IgA antibody having at least bispecificity and comprising a first Fab region containing a first antigen binding site and a second Fab region containing a second antigen binding site.

13. The antibody according to claim 12, which further comprises a secretory component.

14. The antibody according to claim 12, which is a polymer composed of one dimeric IgA antibody molecule containing the first antigen binding site and one or two monomeric IgA antibody molecules containing the second antigen-binding site.

15. The antibody according to claim 13, which is a polymer composed of one dimeric IgA antibody molecule containing the first antigen binding site, one or two monomeric IgA antibody molecules containing the second antigen-binding site, and the secretory component.

16. The antibody according to any one of claims 12 to 15, which comprises four first Fab regions and at least two second Fab regions.

17. The antibody according to any one of claims 13, 15 and 16, wherein the secretory component is a wild-type SC or a mutant SC.

18. A pharmaceutical composition comprising the antibody according to any one of claims 12 to 17.
